# EUROPÄISCHE PATENTSCHRIFT

(11) **EP 1 068 202 B1**
(45) Veröffentlichungstag und Bekanntmachung des Hinweises auf die Patenterteilung: **03.12.2008**
(21) Anmeldenummer: 99915568.2
(22) Anmeldetag: 11.03.1999
(51) Int. Cl.: C07D 413/06, C07D 417/06, C07D 401/06, A61K 31/445

(54) **1-(3 -HETEROARYLPROPYL- ODER -PROP -2-ENYL) -4-BENZYLPIPERIDINE ALS NMDA-REZEPTOR-ANTAGONISTEN**
1-(3-HETEROARYLPROPYL- OR -PROP-2-ENYL)-4-BENZYLPIPERIDINES USED AS NMDA RECEPTOR ANTAGONISTS
1-(3-HETEROARYLPROPYL- OU -PROP-2-ENYL)-4-BENZYLPIPERIDINES UTILISEES COMME ANTAGONISTES DU RECEPTEUR DE NMDA

(30) Priorität: 20.03.1998 DE 19812331
(43) Veröffentlichungstag der Anmeldung: 17.01.2001
(73) Patentinhaber: Merck Patent GmbH, 64293 Darmstadt (DE)
(72) Erfinder: PRÜCHER, Helmut, D-64646 Heppenheim (DE); BARBER, Andrew, D-64331 Weiterstadt (DE); LEIBROCK, Joachim, D-64319 Pfungstadt (DE); BARTOSZYK, Gerd, D-64331 Weiterstadt (DE)
(86) Internationale Anmeldenummer: PCT/EP1999/001573
(87) Internationale Veröffentlichungsnummer: WO 1999/048891

(56) Entgegenhaltungen:
- EP-A- 0 648 744
- EP-A- 0 709 384
- WO-A-96/02250
- WO-A-97/23216
- DE-A- 19 643 790
- FR-A- 2 477 542

## Beschreibung

Die Erfindung betrifft Piperidinderivate ausgewählt aus der Gruppe
a) 6-{3-[4-(4-Fluorobenzyl)-piperidin-1-yl]-propenyl}-3*H-*benzoxazol-2-on;
b) 6-{3-[4-(4-Fluorobenzyl)-piperidin-1-yl]-propyl}-3*H*-benzoxazol-2-on;
c) 6-{3-[4-(4-Fluorobenzyl)-piperidin-1-yl]-propenyl}-3*H-*benzothiazol-2-on;
d) 5-{3-[4-(4-Fluorobenzyl)-piperidin-1-yl]-propenyl}-1,3-dihydrobenzimidazol-2-on,
e) 5-{3-[4-(4-Fluorobenzyl)-piperidin-1-yl]-propenyl}-1,3-dihydroindol-2-on;
sowie deren physiologisch unbedenklichen Salze.

Andere Piperidinderivate kennt man als NMDA-Rrezeptorantagonisten aus der WO 97/23216.
Wiederum andere Piperidinderivate mit regulatorischer Funktion des Zentralnervensystems als auch Antihistaminwirkung sind in FR 2 477 542 beschrieben.

Benzylpiperidinderivate mit hoher Affinität zu Bindungsstellen von Aminosäure-Rezeptoren sind z.B. aus der EP 0 709 384 A1 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die erfindungsgemäßen Verbindungen und ihre Salze bei guter Verträglichkeit sehr wertvolle pharmakologische Eigenschaften besitzen. Vor allem zeigen sie eine besonders hohe Affinität zu Bindungsstellen von Aminosäure-Rezeptoren, insbesondere zur Ifenprodil-Bindungsstelle am NMDA-Rezeptor (NMDA = N-Methyl-D-aspartat), die die Polyamin-Bindungsstelle allosterisch moduliert.
Der Bindungstest für [³H]-Ifenprodil kann nach der Methode von Schoemaker et al., Eur. J. Pharmacol. 176, 249-250 (1990) durchgeführt werden. Die Verbindungen eignen sich zur Behandlung von neurodegenerativen Erkrankungen einschließlich cerebrovaskulären Krankheiten. Ebenso können die neuen Verbindungen als Analgetikum oder Anxiolytikum sowie zur Behandlung von Epilepsie, Schizophrenie, der Alzheimer-, der Parkinson- bzw. der Huntington-Krankheit, cerebralen Ischämien oder Infarkten verwendet werden. Ferner eignen sie sich zur Behandlung von Psychosen, bedingt durch überhöhte Aminosäurespiegel.

Der [³H]-CGP-39653-Bindungstest für die Glutamat-Bindungsstelle des NMDA-Rezeptors kann beispielsweise nach der Methode von M.A.Stills et al., beschrieben in Eur. J. Pharmacol. 192, 19-24 (1991), durchgeführt werden. Der Test für die Glycin-Bindungsstelle des NMDA-Rezeptors ist durchführbar nach der Methode von M.B. Baron et al., beschrieben in Eur. J. Pharmacol. 206, 149-154 (1991).

Die Wirkung gegen Morbus Parkinson, d.h. die Potenzierung des L-DOPAinduzierten kontralateralen Drehens bei hemiparkinsonischen Ratten, ist nach der Methode von U. Ungerstedt und G.W. Arbuthnott, Brain Res. 24, 485 (1970) nachweisbar.

Besonders geeignet ist die Verbindung zur Behandlung oder Prophylaxe von Schlaganfällen sowie zum Schutz vor und zur Behandlung von Hirnödemen und Unterversorgungszuständen des Zentralnervensystems, vor allem Hypoxie oder Anoxie.

Die genannten Wirkungen können außerdem nach den Methoden nachgewiesen oder überprüft werden, wie sie in den folgenden Literaturstellen beschrieben sind:
J.W. McDonald, F.S. Silverstein und M.V. Johnston, Eur. J. Pharmacol. 140, 359 (1987); R. Gill, A.C. Foster und G.N. Woodruff, J. Neurosci. 7, 3343 (1987); J.B. Bederson et al., Stroke, 17, 472-476 (1986); S. Brint et al., J. Cereb. Blood Flow Metab. 8, 474-485 (1988).

Aus den nachfolgend aufgeführten Literaturstellen sind verschiedene Antagonisten bekannt, die verschiedene Bindungsstellen des NMDA-Rezeptors blockieren können:
W. Danysz, C.G. Parsons, I. Bresink und G. Quack, Drug, News & Perspectives 8, 261 (1995), K.R. Gee, Exp. Opin. Invest. Drugs 3, 1021 (1994) und J.J. Kulagowski und L.L. Iversen, J. Med. Chem. 37, 4053 (1994).
Ifenprodil und Eliprodil der Formeln IIIa bzw. IV können den NMDA-Rezeptor blockieren, indem sie eine Wechselwirkung mit der modulatorischen Polyamin-Bindungsstelle eingehen (C.J. Carter, K.G.Lloyd, B. Zivkovic und B. Scatton, J. Pharmacol. Exp. Ther. 253, 475 (1990)).

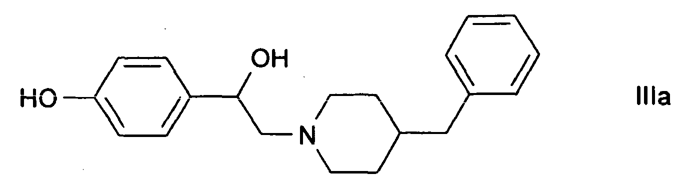

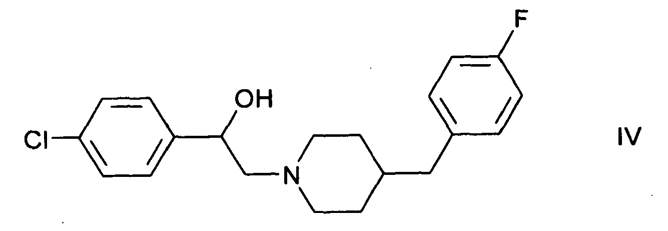

Da Ifenprodil und Eliprodil mit der Polyamin-Bindungsstelle am NMDA-Rezeptor wechselwirken, kann die antagonistische Aktivität der erfindungsgemäßen Verbindungen in einem Spermin-stimulierten [³H]MK-801 (Dizocilpine) - Bindungstest ermittelt werden.
In der Gegenwart von Sättigungskonzentrationen von Glycin und NMDA, kann Spermin noch die Bindung von MK-801 erhöhen, die durch Ifenprodil, Eliprodil und ganz besonders wirksam durch die erfindungsgemäßen Verbindungen inhibiert wird.

Zusätzlich können die erfindungsgemäßen Verbindungen in einem [³H]GABA (y-Aminobuttersäure) - Freisetzungstest, analog J. Dreijer, T. Honoré und A. Schousboe, J. Neurosci. 7, 2910 (1987), der als in-vitro-Modell die antagonistische Funktion in der Zelle beschreibt, getestet werden.

Gegenstand der Erfindung sind demgemäß die Verbindungen nach Anspruch 1 und/oder ihre physiologisch unbedenklichen Salze als Antagonisten an Rezeptoren von exzitatorischen Aminosäuren, wie z.B. Glutaminsäure bzw. deren Salze.
Gegenstand der Erfindung sind insbesondere die Verbindungen nach Anspruch 1 und/oder ihrer unbedenklichen Salze, als exzitatorische Aminosäuren-Antagonisten zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

Gegenstand der Erfindung ist auch die Verwendung der Verbindungen nach Anspruch 1 und/oder ihrer physiologisch unbedenklichen Salze zur Herstellung eines Arzneimittels zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Eine erfindungsgemäße Base kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung ist ferner die Verwendung der erfindungsgemäßen Verbindungen und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend eine wirksame Menge mindestens einer der erfindungsgemäßen Verbindungen und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.
Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die erfindungsgemäßen Verbindungen und ihre physiologisch unbedenklichen Salze können als exzitatorische Aminosäure-Antagonist bei der Bekämpfung von Krankheiten, insbesondere zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen verwendet werden.

Dabei können die erfindungsgemäßen Verbindungen in der Regel in Analogie zu anderen bekannten Verbindungen mit ähnlichem Wirkprofil, wie z.B. Ifenprodil, verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von, der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1

Eine Suspension von 5,5 g 6-(3-Chlorpropyl)-3H-benzoxazol-2-on in 50 ml Ethanol wird mit 5,7 g 4-(4-Fluorbenzyl)-piperidin Hydrochlorid und 7,2 ml Triethylamin versetzt. Man rührt eine Stunde unter Rückfluß und arbeitet wie üblich auf und erhält 8,5 g 6-{3-[4-(4-Fluorobenzyl)-piperidin-1-yl]-propyl}-3*H*-benzoxazol-2-on, F. 105-107°.

### Beispiel 2

Eine Lösung aus 6,7 g 6-{3-[4-(4-Fluorobenzyl)-piperidin-1-yl]-1-hydroxypropyl}-3*H*-benzoxazol-2-on [erhältlich durch Hydrierung von 6-{3-[4-(4-Fluorobenzyl)-piperidin-1-yl]-1-oxo-propyl}-3*H-*benzoxazol-2-on, das durch Umsetzung von 4-(4-Fluorbenzyl)-piperidin und 6-(3-Chloro-propionyl)-3*H-*benzoxazol-2-on zugänglich ist] in 70 ml Dioxan wird mit 7 ml konz. HCl 1,5 h unter Rückfluß erhitzt. Man kühlt ab und gibt 8,5 g NaHCO₃ und 70 ml Wasser zu. Man setzt 30 ml Dichlormethan zu und rührt 30 Minuten nach. Man trennt den Niederschlag ab, wäscht mit Aceton und Ether, trocknet und erhält 5,7 g 6-{3-[4-(4-Fluorobenzyl)-piperidin-1-yl]-propenyl}-3*H-*benzoxazol-2-on ("A"), F. 202-203,5°; Hydrochlorid: F. 220-223°.

Analog erhält man die Verbindungen
6-{3-[4-(4-Fluorobenzyl)-piperidin-1-yl]-propenyl)-3*H*-benzothiazol-2-on, Hydrochlorid x H₂O, F. 95-99° (Zersetzung);
5-{3-[4-(4-Fluorobenzyl)-piperidin-1-yl]-propenyl}-1,3-dihydrobenzimidazol-2-on, F. 218-220°; Hydrochlorid: 243-245°.
5-{3-[4-(4-Fluorobenzyl)-piperidin-1-yl]-propenyl}-1,3-dihydro-indol-2-on.

### Beispiel 3

Eine Lösung aus 2,56 g "A" in 100 ml Methanol und 100 ml THF wird unter Zugabe von 1,2 g Pd/C bei Raumtemperatur hydriert. Man trennt den Katalysator ab, arbeitet wie üblich auf und erhält 1,31 g 6-{3-[4-(4-Fluorobenzyl)-piperidin-1-yl]-propyl}-3*H*-benzoxazol-2-on, F. 105-107°.

Analog erhält man durch Hydrierung
aus 6-{3-[4-(4-Fluorobenzyl)-piperidin-1-yl]-propenyl}-3*H*-benzothiazol-2-on 6-{3-[4-(4-Fluorobenzyl)-piperidin-1-yl]-propyl}-3*H*-benzothiazol-2-on;
aus 5-{3-[4-(4-Fluorobenzyl)-piperidin-1-yl]-propenyl}-1,3-dihydrobenzimidazol-2-on
5-{3-[4-(4-Fluorobenzyl)-piperidin-1-yl]-propyl}-1,3-dihydrobenzimidazol-2-on;
aus 5-(3-[4-(4-Fluorobenzyl)-piperidin-1-yl]-propenyl)-1,3-dihydro-indol-2-on
5-{3-[4-(4-Fluorobenzyl)-piperidin-1-yl]-propyl}-1,3-dihydro-indol-2-on. re oder THF durchgeführt, man kann auch Gemische dieser Lösungsmittel einsetzen.

Verbindungen der Formel I worin Y und Z zusammen C=C bedeuten, können vorzugsweise aus Verbindungen der Formel I erhalten werden, worin
- X, R¹, R³ und R⁴: die in Anspruch 1 angegebenen Bedeutungen haben,
- Y: CH,
- Z: CH und
- R²: OH oder L' ist, worin L' Cl, Br, I, OH oder eine reaktionsfähig veresterte OH-Gruppe bedeutet,
indem man aus diesen Verbindungen Wasser oder L'H abspaltet.

Die Abspaltung erfolgt vorzugsweise mit wässrigen Säuren, insbesondere wässrigen Mineralsäuren.

L' bedeutet z.B. vorzugsweise Cl, Br, I oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Verbindungen, in denen R² OH oder L' ist, lassen sich durch Reduktion z.B. aus solchen Verbindungen erhalten, in denen Y und R² zusammen eine Carbonylgruppe bilden.
Die Reduktion kann wie angegeben durch katalytische Hydrierung oder mit komplexen Metallhydriden erfolgen.

Als komplexe Metallhydride können z.B. NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether, wie Diethylether, Di-n-butylether, THF, Dioxan, Diglyme oder 1,2-Dimethoxyethan, sowie Kohlenwasserstoffe, wie Benzol. Für eine Reduktion mit NaBH₄ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150 °C, insbesondere zwischen 0 und etwa 100 °C.

Die Umwandlung einer OH-Gruppe in eine OL'-Gruppe erfolgt nach bekannten und üblichen Methoden.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend eine wirksame Menge mindestens einer der Verbindungen der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder lmplantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.
Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die Verbindungen der Formel I und ihre physiologisch unbedenklichen Salze können als exzitatorische Aminosäure-Antagonist bei der Bekämpfung von Krankheiten, insbesondere zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen verwendet werden.

Dabei können die erfindungsgemäßen Verbindungen in der Regel in Analogie zu anderen bekannten Verbindungen mit ähnlichem Wirkprofil, wie z.B. Ifenprodil, verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1

Eine Suspension von 5,5 g 6-(3-Chlorpropyl)-3H-benzoxazol-2-on in 50 ml Ethanol wird mit 5,7 g 4-(4-Fluorbenzyl)-piperidin Hydrochlorid und 7,2 ml Triethylamin versetzt. Man rührt eine Stunde unter Rückfluß und arbeitet wie üblich auf und erhält 8,5 g 6-{3-[4-(4-Fluorobenzyl)-piperidin-1-yl]-propyl}-3*H*-benzoxazol-2-on, F. 105-107°.

### Beispiel 2

Eine Lösung aus 6,7 g 6-{3-[4-(4-Fluorobenzyl)-piperidin-1-yl]-1-hydroxypropyl}-3*H*-benzoxazol-2-on [erhältlich durch Hydrierung von 6-{3-[4-(4-Fluorobenzyl)-piperidin-1-yl]-1-oxo-propyl}-3*H*-benzoxazol-2-on, das durch Umsetzung von 4-(4-Fluorbenzyl)-piperidin und 6-(3-Chloro-propionyl)-3*H-*benzoxazol-2-on zugänglich ist] in 70 ml Dioxan wird mit 7 ml konz. HCl 1,5 h unter Rückfluß erhitzt. Man kühlt ab und gibt 8,5 g NaHCO₃ und 70 ml Wasser zu. Man setzt 30 ml Dichlormethan zu und rührt 30 Minuten nach. Man trennt den Niederschlag ab, wäscht mit Aceton und Ether, trocknet und erhält 5,7 g 6-{3-[4-(4-Fluorobenzyl)-piperidin-1-yl]-propenyl}-3*H*-benzoxazol-2-on ("A"), F. 202-203,5°; Hydrochlorid: F. 220-223°.

Analog erhält man die Verbindungen
6-{3-[4-(4-Fluarobenzyl)-piperidin-1-yl]-2-methyl-propenyl}-3*H-*benzoxazol-2-on, F. 156-160°, Hydrochlorid: 230-235°;
6-{3-[4-(4-Fluorobenzyl)-piperidin-1-yl]-propenyl}-3*H*-benzothiazol-2-on, Hydrochlorid x H₂O, F. 95-99° (Zersetzung);
5-{3-[4-(4-Fluorobenzyl)-piperidin-1-yl]-propenyl}-1,3-dihydrobenzimidazol-2-on, F. 218-220°; Hydrochlorid: 243-245°.
5-{3-[4-(4-Fluorobenzyl)-piperidin-1-yl]-propenyl}-1,3-dihydro-indol-2-on;
6-{3-[4-(4-Fluorobenzyl)-piperidin-1-yl]-propenyl}-3,4-dihydro-1*H-*chinolin-2-on.

### Beispiel 3

Eine Lösung aus 2,56 g "A" in 100 ml Methanol und 100 ml THF wird unter Zugabe von 1,2 g Pd/C bei Raumtemperatur hydriert. Man trennt den Katalysator ab, arbeitet wie üblich auf und erhält 1,31 g 6-{3-[4-(4-Fluorobenzyl)-piperidin-1-yl]-propyl}-3*H*-benzoxazol-2-on, F. 105-107°.

Analog erhält man durch Hydrierung
aus 6-{3-[4-(4-Fluorobenzyl)-piperidin-1-yl]-2-methyl-propenyl}-3*H-*benzoxazol-2-on
6-{3-[4-(4-Fluorobenzyl)-piperidin-1-yl]-2-methyl-propyl}-3*H-*benzoxazol-2-on;
aus 6-{3-[4-(4-Fluorobenzyl)-piperidin-1-yl]-propenyl}-3*H*-benzothiazol-2-on 6-{3-[4-(4-Fluorobenzyl)-piperidin-1-yl]-propyl}-3*H*-benzothiazol-2-on;
aus 5-{3-[4-(4-Fluorobenzyl)-piperidin-1-yl]-propenyl}-1,3-dihydrobenzimidazol-2-on
5-{3-[4-(4-Fluorobenzyl)-piperidin-1-yl]-propyl}-1,3-dihydrobenzimidazol-2-on;
aus 5-{3-[4-(4-Fluorobenzyl)-piperidin-1-yl]-propenyl}-1,3-dihydro-indol-2-on
5-{3-[4-(4-Fluorobenzyl)-piperidin-1-yl]-propyl}-1,3-dihydro-indol-2-on
aus 6-{3-[4-(4-Fluorobenzyl)-piperidin-1-yl]-propenyl}-3,4-dihydro-1*H-*chinolin-2-on
6-{3-[4-(4-Fluorobenzyl)-piperidin-1-yl]-propyl}-3,4-dihydro-1*H-*chinolin-2-on.

Die nachfolgenden Beispiele betreffen pharmazeutische Zubereitungen:

### Beispiel A: Injektionsgläser

Eine Lösung von 100 g des Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g des Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g des Wirkstoffes der Formel I, 9,38 g NaH₂PO₄ 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg des Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen ausgewählt aus der Gruppe
a) 6-{3-[4-(4-Fluorobenzyl)-piperidin-1-yl]-propenyl}-3*H*- benzoxazol-2-on;
b) 6-{3-[4-(4-Fluorobenzyl)-piperidin-1-yl]-propyl}-3*H*-benzoxazol-2-on;
c) 6-{3-[4-(4-Fluorobenzyl)-piperidin-1-yl]-propenyl}-3*H*- benzothiazol-2-on;
d) 5-{3-[4-(4-Fluorobenzyl)-piperidin-1-yl]-propenyl}-1,3-dihydrobenzimidazol-2-on,
e) 5-{3-[4-(4-Fluorobenzyl)-piperidin-1-yl]-propenyl}-1,3-dihydroindol-2-on;
sowie deren physiologisch unbedenklichen Salze.

2. Verfahren zur Herstellung einer pharmazeutischen Zubereitung, **dadurch gekennzeichnet, daß** man eine Verbindung nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

3. Pharmazeutische Zubereitung, **gekennzeichnet durch** einen wirksamen Gehalt an mindestens einer Verbindung nach Anspruch 1 und/oder einem ihrer physiologisch unbedenklichen Salze.

4. Verbindungen nach Anspruch 1 und/oder ihre physiologisch unbedenklichen Salze als exzitatorische Aminosäuren-Antagonisten.

5. Verbindungen nach Anspruch 1 und ihre physiologisch unbedenklichen Salze als exzitatorische Aminosäuren-Antagonisten zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

6. Verwendung der Verbindungen nach Anspruch 1 und/oder ihre physiologisch unbedenklichen Salze zur Herstellung eines Arzneimittels zur Bekämpfung von neurodegenerativen Erkrankungen einschließlich cerebrovaskulären Krankheiten, Epilepsie, Schizophrenie, der Alzheimer-, der Parkinson- bzw. der Huntington-Krankheit, cerebralen Ischämien, Infarkten oder Psychosen.

## Claims

1. Compounds selected from the group
a) 6-{3-[4-(4-fluorobenzyl)piperidin-1-yl]propenyl}-3*H*-benzoxazol-2-one;
b) 6-{3-[4-(4-fluorobenzyl)piperidin-1-yl]propyl}-3*H*-benzoxazol-2-one;
c) 6-{3-[4-(4-fluorobenzyl)piperidin-1-yl]propenyl}-3*H*-benzothiazol-2-one;
d) 5-{3-[4-(4-fluorobenzyl)piperidin-1-yl]propenyl}-1,3-dihydrobenzimidazol-2-one;
e) 5-{3-[4-(4-fluorobenzyl)piperidin-1-yl]propenyl}-1,3-dihydroindol-2-one;
and physiologically acceptable salts thereof.

2. Process for the preparation of a pharmaceutical composition, **characterised in that** a compound according to Claim 1 and/or one of its physiologically acceptable salts is converted into a suitable dosage form together with at least one solid, liquid or semi-liquid excipient or adjuvant.

3. Pharmaceutical composition, **characterised by** an effective content of at least one compound according to Claim 1 and/or one of its physiologically acceptable salts.

4. Compounds according to Claim 1 and/or physiologically acceptable salts thereof as excitatory amino acid antagonists.

5. Compounds according to Claim 1 and physiologically acceptable salts thereof as excitatory amino acid antagonists for combating neurodegenerative diseases, including cerebrovascular diseases, epilepsy, schizophrenia, Alzheimer's, Parkinson's or Huntington's disease, cerebral ischaemia, infarctions or psychoses.

6. Use of the compounds according to Claim 1 and/or physiologically acceptable salts thereof for the preparation of a medicament for combating neurodegenerative diseases, including cerebrovascular diseases, epilepsy, schizophrenia, Alzheimer's, Parkinson's or Huntington's disease, cerebral ischaemia, infarctions or psychoses.

## Revendications

1. Composés sélectionnés parmi le groupe
a) la 6-{3-[4-(4-fluorobenzyl)pipéridin-1-yl]propényl}-3*H*-benzoxazol-2-one ;
b) la 6-{3-[4-(4-fluorobenzyl)pipéridin-1-yl]propyl}-3*H*-benzoxazol-2-one ;
c) la 6-{3-[4-(4-fluorobenzyl)pipéridin-1-yl]propényl}-3*H*-benzothiazol-2-one ;
d) la 5-{3-[4-(4-fluorobenzyl)pipéridin-1-yl]propényl}-1,3-dihydrobenzimidazol-2-one ;
e) la 5-{3-[4-(4-fluorobenzyl)pipéridin-1-yl]propényl}-1,3-dihydroindol-2-one ;
et les sels physiologiquement acceptables de ceux-ci.

2. Procédé de préparation d'une composition pharmaceutique, **caractérisé en ce qu'**un composé selon la revendication 1 et/ou l'un de ses sels physiologiquement acceptables est converti en une forme de dosage appropriée conjointement avec au moins un excipient ou adjuvant solide, liquide ou semi-liquide.

3. Composition pharmaceutique, **caractérisée par** une teneur efficace en au moins un composé selon la revendication 1 et/ou en l'un de ses sels physiologiquement acceptables.

4. Composés selon la revendication 1 et/ou des sels physiologiquement acceptables de ceux-ci comme antagonistes d'acides aminés excitateurs.

5. Composés selon la revendication 1 et des sels physiologiquement acceptables de ceux-ci comme antagonistes d'acides aminés excitateurs destinés à lutter contre des maladies neurodégénératives, y compris les maladies cérébrovasculaires, l'épilepsie, la schizophrénie, la maladie d'Alzheimer, de Parkinson ou de Huntington, l'ischémie cérébrale, les infarctus ou les psychoses.

6. Utilisation des composés selon la revendication 1 et/ou de sels physiologiquement acceptables de ceux-ci pour la préparation d'un médicament destiné à lutter contre des maladies neurodégénératives, y compris les maladies cérébrovasculaires, l'épilepsie, la schizophrénie, la maladie d'Alzheimer, de Parkinson ou de Huntington, l'ischémie cérébrale, les infarctus ou les psychoses.
